# EUROPEAN PATENT APPLICATION

(11) **EP 1 625 873 A1**
(43) Date of publication of application: **15.02.2006**
(21) Application number: 04733455.2
(22) Date of filing: 17.05.2004
(51) Int. Cl.: A61M 39/02, A61J 1/20

(54) **SEALING BODY, CAP WITH THE SEALING BODY, AND MEDICAL CONTAINER**

(30) Priority: 22.05.2003 JP 2003145250
(71) Applicant: OTSUKA PHARMACEUTICAL FACTORY, INC., Naruto-shi, Tokushima 772-8601 (JP)
(72) Inventor: Tezuka, Kenji, Naruto-shi, Tokushima 779-0234 (JP); Honda, Hiroshi, Tokushima-shi, Tokushima 770-0024 (JP); Kaga, Junji, Itano-gun, Tokushima 7710205 (JP); Oka, Minoru, Itano-gun, Tokushima 7710205 (JP); Shoji, Hidekatsu, Itano-gun, Tokushima 771-0207 (JP)
(74) Representative: HOFFMANN EITLE
(86) International application number: PCT/JP2004/006988
(87) International publication number: WO 2004/103453

(57) **Abstract**

An inventive sealing body is a generally disk-like sealing body composed of two or more types of thermoplastic elastomers having different hardnesses, and comprises at least a needle stick part extended in axial direction and an outer edge part surrounding the needle stick part. The outer edge part has a higher hardness than that of the needle stick part. More specifically, a difference in JIS-A hardness between the outer edge part and the needle stick part, for example, is 5 to 15. The sealing body is tightly fitted in a generally cylindrical outer frame body composed of a rigid plastic to provide a cap. The cap is welded to a mouth part of a plastic container or the mouth part is tightly sealed with the sealing body to provide a medical container.

## Description

### TECHNICAL FIELD

The present invention relates to a sealing body to be used for sealing a mouth part of a medical container such as an infusion bottle, an infusion bag or a vial, and a cap and a medical container each including such a sealing body.

### BACKGROUND ART

An elastic material is used as a sealing body for sealing a mouth part of a medical container such as an infusion bottle, an infusion bag or a vial for containing a drug solution or a drug in consideration of the fact that the sealing body is stuck with a plastic sticking member(hollow needle) having a relatively great diameter. In general, a rubber is used as the elastic material. In recent years, a thermoplastic elastomer, which is advantageous in that it has a good moldability and little bleeding, has been used instead of the rubber (see Japanese Unexamined Utility Model Publication No. SH060(1985)-147433).

For improvement of the sealability of the sealing body during the sticking with the sticking member, the sealing body should have a lower hardness. However, if the sealing body is composed of a thermoplastic elastomer having a lower hardness, the sealing body is more liable to warp during the sticking with the sticking member, resulting in handling difficulty. Further, the sticking member is more liable to come off from the sealing body due to reduction of the sticking member holding capability of the sealing body. Furthermore, the thermoplastic elastomer having a lower hardness has a higher tackiness, so that sealing bodies are more liable to adhere to each other. This hinders smooth flow of the sealing bodies in a production line.

On the other hand, if the sealing body is composed of a thermoplastic elastomer having a higher hardness, it is difficult to stick the sealing body with the sticking member. Further, the sealing body has a reduced sealability during the sticking with the sticking member or after withdrawal of the sticking member, thereby causing liquid leakage from the medical container.

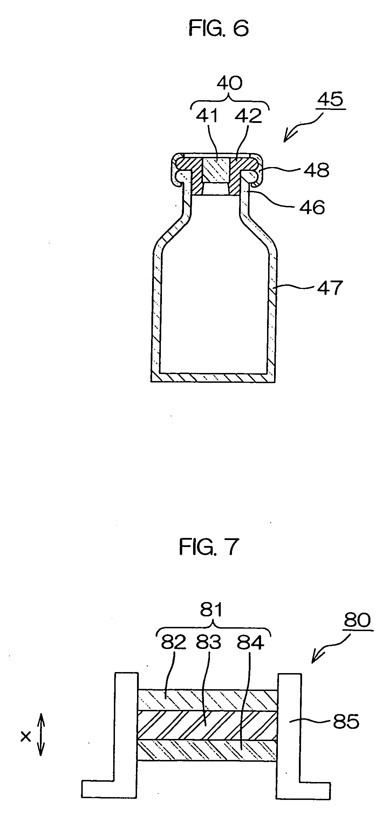

Japanese Unexamined Patent Publication No. HEI11 (1999)-19177 discloses a generally columnar sealing body 81 as shown in Fig. 7, which includes thermoplastic elastomer thin layers 82 to 84 stacked one on another, wherein an upper one and a lower one of the thin layers have different elastic moduli. In Fig. 7, a reference character 80 denotes a cap, and a reference character 85 denotes an outer frame body.

However, even if the thermoplastic elastomer sealing body has the aforesaid layer structure, it is insufficient to solve the technical problems described above. In addition, it is impossible to alleviate the problemof the adhesion (tackiness) of the sealingbodies.

### DISCLOSURE OF THE INVENTION

It is an object of the present invention to provide a thermoplastic elastomer sealing body which simultaneously ensures easier sticking with a sticking member and a sufficient sealability during the sticking as well as a sufficient sticking member holding capability and easier handling during the sticking, and is free from adhesion to other sealing bodies.

It is another object of the present invention to provide a cap and a medical container each including the aforesaid sealing body.

To solve the aforesaid problems, the present invention provides a sealing body having a generally disk-like shape formed of two or more types of thermoplastic elastomers with different hardnesses, the sealing body comprising at least a needle stick part extended in axial direction and an outer edge part surrounding the needle stick part, the outer edge part having a higher hardness than that of the needle stick part.

As described above, the inventive sealing body has at least two types of regions including the needle stick part and the outer edge part surrounding the needle stick part. Even if the thermoplastic elastomer for the needle stick part has a lower hardness to ensure easier sticking of the sealing body with a sticking member and sufficient sealability of the sealing body during the sticking, it is possible to ensure easier handling during the sticking of the sealing body with the sticking member and a sufficient sticking member holding capability because the thermoplastic elastomer for the outer edge part surrounding the needle stick part has a higher hardness. Therefore, the easier sticking with the sticking member and the sufficient sealability during the sticking as well as the sufficient stickingmember holding capability and the easier handling during the sticking can be simultaneouslyensured. Inaddition, the outer edge part of the sealing body has a reduced adhesiveness because the thermoplastic elastomer for the outer edge part has a higher hardness. Hence, the adhesion between sealing bodies can be prevented. Therefore, the inventive sealing body is advantageously used as a sealing body for sealing a mouth part of a medical container such as an infusion bottle, an infusion bag or a vial.

In the inventive sealing body, the outer edge part preferably has a JIS A hardness of 25 to 80, and the needle stick part preferably has a JIS A hardness of 5 to 30. Where the outer edge part and the needle stick part respectively have hardnesses within the aforesaid hardness ranges and the hardness of the outer edge part is higher than the hardness of the needle stick part, the ease of sticking with the sticking member, the sealability during the sticking, the sticking member holding capability and the ease of handling during the sticking can be further improved. In addition, the adhesion between sealing bodies can further assuredly be prevented.

In the present invention, "JIS A hardness" is a hardness Hs (JIS A) determined by a spring hardness test (Type A) specified by "5. Hardness Test" in Japanese Industrial Standard JIS K 6301₋₁₉₇₅ (Physical Test Methods for vulcanized Rubbers).

In the inventive sealing body, the needle stick part of the thermoplastic elastomer having a lower hardness may include a single needle stick part or a plurality of needle stick parts, but the number of the needle stick parts is not particularly limited.

Where the single needle stick part is provided in the sealing body, the needle stick part is preferably provided in a center portion of the sealing body and has a cylindrical shape. Further, the needle stick part preferablyhas a diameter which is 40 to 70% of the diameter of the entire sealing body. With the diameter of the needle stick part being within the aforesaid percentage range with respect to the diameter of the entire sealing body, a sufficient sealability during the sticking as well as a sufficient sticking member holding capability and easier handling during the sticking can further assuredly be provided.

The inventive sealing body may include two to five needle stick parts. With the provision of the plurality of needle stick parts (e.g., the two to five needle stick parts), the sealing body can be simultaneously stuck with a sticking needle for injecting a drug solution or air into a medical container having the sealing body and with a sticking needle for discharging a drug solution or air from the medical container, and a sticking position can be changed depending on the size and type of the sticking member to be used. Therefore, the sealing body, and a cap and a medical container each including the sealing body can be advantageously used and handled.

To solve the aforesaid problems, the present invention provides a cap which comprises the afore-said inventive sealing body being tightly fitted in a generally cylindrical outer flame body formed of a rigid plastic. That is, examples of the cap include a cap comprising a sealing body having a generally disk-like shape formed of two or more types of thermoplastic elastomers with different hardnesses, the sealing body comprising at least a needle stick part extended in axial direction and an outer edge part surrounding the needle stick part and having a higher hardness than that of the needle stick part, wherein the sealing body is tightly fitted in an outer frame body having a generally cylindrical shape formed of a rigid plastic.

By tightly fitting the inventive sealing body in the generally cylindrical outer frame body formed of the rigid plastic, the inventive sealing body can be easily applied to a mouth part of a plastic medical container such as an infusion bottle or an infusion bag.

To solve the aforesaid problems, the present invention also provides the following medical containers.
(I) A medical container made of plastic comprising the aforesaid inventive cap which is welded to a mouth part of the medical container.
   That is, examples of the medical container include a medical container made of plastic comprising a cap which is welded to a mouth part of the medical container, the cap comprising a sealing body having a generally disk-like shape formed of two or more types of thermoplastic elastomers with different hardnesses, the sealing body comprising at least a needle stick part extended in axial direction and an outer edge part surrounding the needle stick part and having a higher hardness than that of the needle stick part, wherein the sealing body is tightly fitted in an outer frame body having a generally cylindrical shape formed of a rigid plastic.
(II) A medical container comprising a mouth part,
   wherein the mouth part is tightly sealed by the aforesaid inventive sealing body

That is, examples of the medical container include a medical container comprising a mouth part, wherein the mouth part is tightly sealed by a sealing body having a generally disk-like shape formed of two or more types of thermoplastic elastomers with different hardnesses, the sealing body comprising at least a needle stick part extended in axial direction and an outer edge part surrounding the needle stick part and having a higher hardness than that of the needle stick part.

The medical container (I) is such that the inventive cap including the outer frame body formed of the rigid plastic is provided in the mouth part. Therefore, the mouth part of the medical container per se should be weldable to the outer frame body of the cap. Specific examples of the medical container (I) include plastic medical containers such as an infusion bottle and an infusion bag.

The medical container (II) is such that the inventive sealing body is fitted in the mouth part with no gap to seal the mouth part. The type of the medical container is not particularly limited, but the inner diameter of the mouth part of the medical container should conform to the outer diameter of the sealing body. This medical container is applicable not only to a plastic medical container such as an infusion bottle or an infusion bag but also to a medical container such as a vial.

### BRIEF DESCRIPTION OF THE DRAWINGS

Figs. 1(a), 1(b) and 1(c) are a plan view, a sectional view and a bottom view, respectively, illustrating a sealing body according to an embodiment of the present invention.
Fig. 2 is a sectional view illustrating a cap including the sealing body 10 of Fig. 1 according to the embodiment.
Fig. 3 is a front view illustrating, partly in section, a medical container (infusion bottle) including the sealing body 10 of Fig. 1 according to the embodiment.
Figs. 4(a), 4(b) and 4(c) are a plan view, a sectional view taken along a line A-A and a bottom view, respectively, illustrating a sealing body according to another embodiment of the present invention.
Figs. 5(a), 5(b) and 5(c) are a plan view, a sectional view and a bottom view, respectively, illustrating a sealing body according to further another embodiment of the present invention.
Fig. 6 is a sectional view illustrating a medical container (ampoule) including the sealing body 40 of Fig. 5 according to the further another embodiment.
Fig. 7 is a sectional view schematically illustrating a cap disclosed in Japanese Unexamined Patent Publication No. HEI11(1999)-19177.

### BEST MODE FOR IMPLEMENTING THE INVENTION

The sealing body, the cap and the medical container according to the present invention will hereinafter be described in detail with reference to the attached drawings.

### First Embodiment

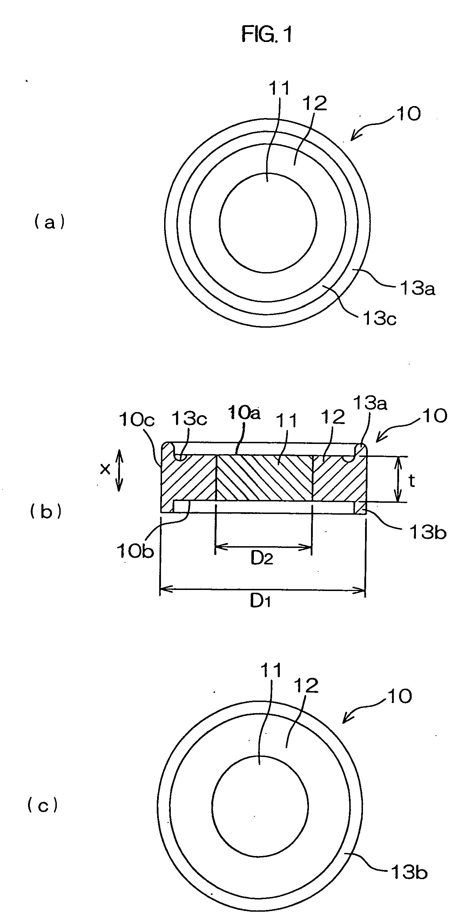

A sealing body 10 shown in Fig. 1 is an exemplary sealing body according to one embodiment of the present invention. The sealing body 10 has a disk shape, and includes a cylindrical needle stick part 11 extending in an axial direction x and an outer edge part 12 surrounding the needle stick part 11.

In the sealing body 10 shown in Fig. 1, a single needle stick part 11 is provided in a center portion of the sealing body 10. In this case, the diameter D2 of the needle stick part 11 is preferably 40 to 70% of the diameter D1 of the entire sealing body 10.

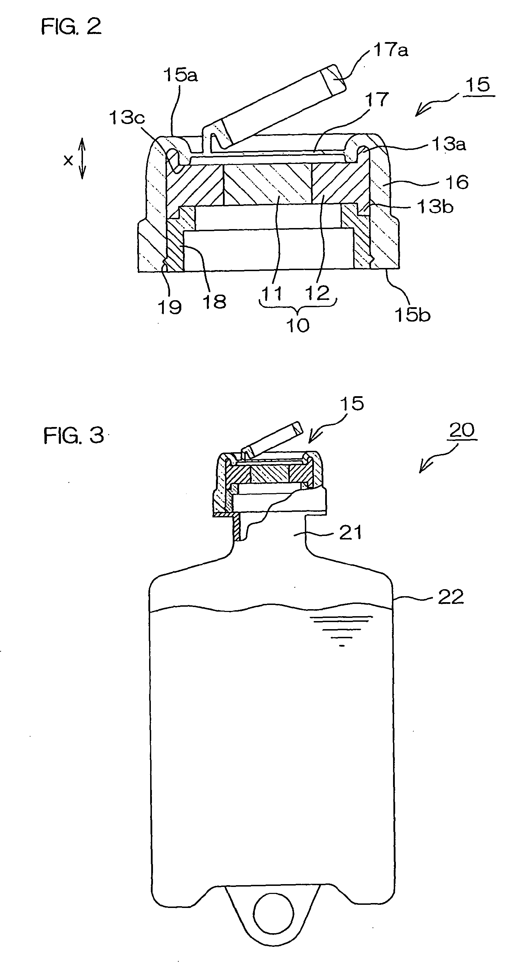

A cap 15 shown in Fig. 2 and an infusion bottle 2'0 shown in Fig. 3 are an exemplary cap and medical container according to the embodiment of the present invention.

The cap 15 shown in Fig. 2 includes the sealing body 10 shown in Fig. 1, a generally cylindrical outer frame body 16 of a rigid plastic, and a generally cylindrical inner frame body 18. The sealing body 10 is inserted in a cylindrical portion of the outer frame body 16 from the side of a bottom 15b thereby to be fitted in the outer frame body 16 with no gap formed between an inner peripheral surface of the outer frame body 16 and an outer peripheral surface 10c of the sealing body 10. The sealing body 10 is fixed in the cylindrical portion of the outer frame body 16 by inserting the inner frame body 18 in the outer frame body 16 from the side of the bottom 15b. Projections 13a, 13b respectively provided on a top surface 10a and a bottom surface 10b of the sealing body 10 and a groove 13c provided in the top surface 10a of the sealing body 10 are engaged with the outer frame body 16 and the inner frame body 18, whereby the sealing body is more assuredly fixed to the cylindrical portion of the outer frame body 16.

The cap 15 shown in Fig. 2 includes a cover 17 of a pull-off (pull-top) type. By pulling up a ring 17a, the cover 17 is pulled off. The cover 17 protects the top surface 10a of the sealing body from dust until the cap 15 is actually used.

The infusion bottle 20 shown in Fig. 3 is such that the cap 15 shown in Fig. 2 is welded to a mouth part 21 of a plastic medical container (medical container body 22).

### Second Embodiment

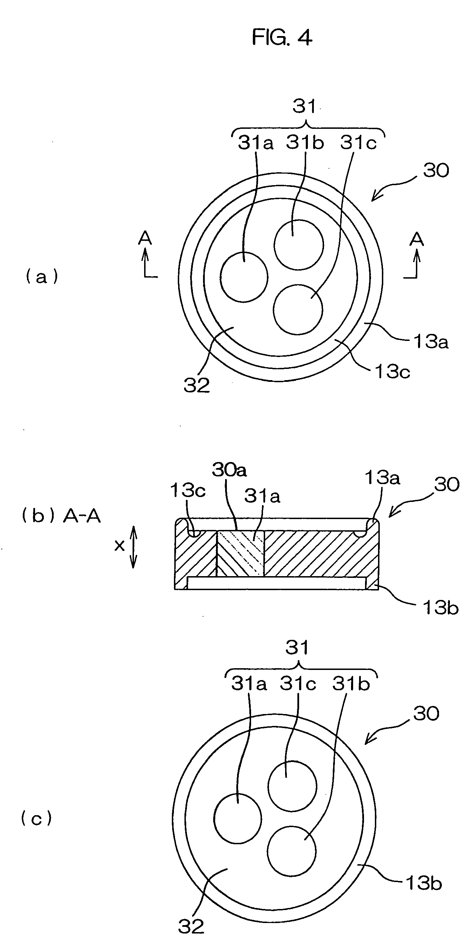

A sealing body 30 shown in Fig. 4 is an exemplary sealing body according to another embodiment of the present invention. Like the sealing body 10 shown in Fig. 1, the sealing body 30 has a disk shape.

The sealing body 30 includes three cylindrical needle stick parts 31 each extending in an axial direction x, and an outer edge part 32 surrounding all the needle stick parts 31a to 31c.

In the sealing body 30 shown in Fig. 4, the ratio of the diameter of each of the needle stick parts 31 to the diameter of the entire sealing body 30 is not particularly limited, but may be properly determined depending on the hardnesses of the needle stick parts 31 and the outer edge part 32 for simultaneously ensuring a sufficient sealability during the sticking as well as a sufficient sticking member holding capability and easier handling during the sticking.

In Fig. 4, components other than those described above are denoted by the same reference characters as in Fig. 1.

### Third Embodiment

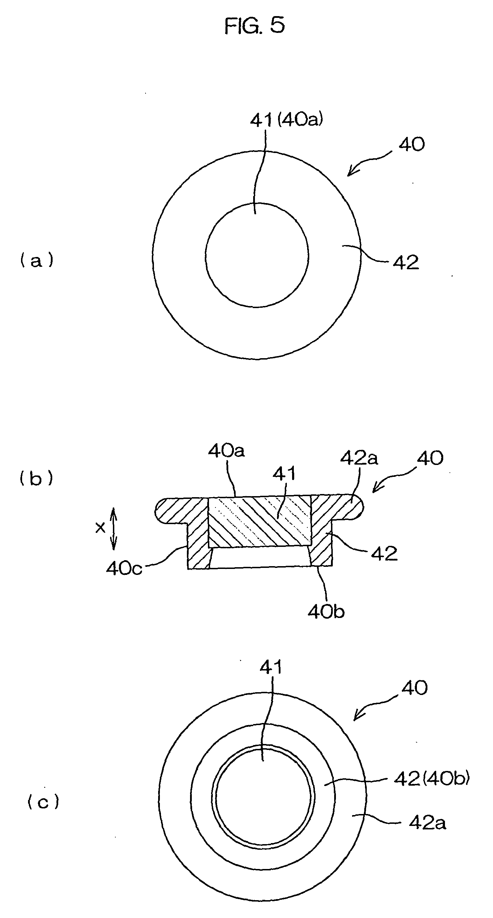

A sealing body 40 shown in Fig. 5 is an exemplary sealing body according to further another embodiment of the present invention. The sealing body 40 includes a cylindrical needle stick part 41 extending in an axial direction x, and an outer edge part 42 surrounding the needle stick part 41. The outer edge part 42 includes a flange 42a provided on an outer peripheral surface 40a of the sealing body 40 for engagement with a mouth part edge of a medical container.

The sealing body 40 shown in Fig. 5 is used for tightly sealing a mouth part 46 of a vial 45 as shown in Fig. 6. The outer shape of the sealing body 40 is properly determined so that no gap is formed between the outer peripheral surface 40a and the mouth part 46 of the vial 45. To firmly fix the sealing body 40 to the vial 45, a clamping member 48 may be provided around the mouth part 46 and the outer edge part of the sealing body 45.

### Sealing body

An essential requirement for the inventive sealing body is that the hardness of the needle stick part is lower than the hardness of the outer edge part. In addition, the needle stick part per se preferably has a JIS A hardness of 5 to 30. If the JIS A hardness of the needle stick part (the hardness of the thermoplastic elastomer for the needle stick part) is lower than 5, it is impossible to ensure a sufficient sticking member holding capability and easier handling during the sticking. Further, the needle stick part has a reduced mechanical strength and, therefore, is more liable to be broken. On the other hand, if the JIS A hardness of the needle stick part (the hardness of the thermoplastic elastomer for the needle stick part) is higher than 30, the ease of the sticking with the sticking member is lessened (the sticking with the sticking member is more difficult) and the sealability during the sticking is deteriorated. Particularly, the JIS A hardness of the needle stick part is preferably not lower than 15 and lower than 30, more preferably not lower than 18 and not higher than 25, in the aforesaid hardness range.

Another essential requirement for the inventive sealing body is that the outer edge part has a higher hardness than the needle stick part. In addition, the outer edge part per se preferably has a JIS A hardness of 25 to 80. If the JIS A hardness of the outer edge part (the hardness of the thermoplastic elastomer for the outer edge part) is lower than 25, it may be impossible to ensure a sufficient sticking member holding capability and easier handling during the sticking. On the other hand, if the JIS A hardness of the outer edge part (the hardness of the thermoplastic elastomer for the outer edge part) is higher than 80, the welding between the outer edge part and the needle stick part is difficult, and the adhesion between the outer frame body and the inner frame body is reduced. Particularly, the JIS A hardness of the outer edge part is preferably 30 to 45 in the aforesaid hardness range.'

In the inventive sealing body, a difference in JIS A hardness between the needle stick part and the outer edge part is not particularly limited, but is preferably about 5 to about 15.

In the inventive sealing body, the thermoplastic elastomers for the needle stick part and the outer edge part are merely required to be weldable to each other, but the other requirements for these thermoplastic elastomers are not particularly limited. For improvement of the weldability, thermoplastic elastomers of the same type are preferably used.

Examples of the thermoplastic elastomers usable in the present invention include conventionally known various thermoplastic elastomers including styrene elastomers such as a styrene-ethylene/butylene-styrene block copolymer (SEBS), a styrene-butadiene-styrene block copolymer (SBS), a styrene-isoprene-styrene block copolymer (SIS), a SEBS modified with maleic acid or the like, a styrene-ethylene/propylene-styrene block copolymer (SEPS), a styrene-ethylene/butylene block copolymer (SEB) and a styrene-ethylene/propylene block copolymer (SEP), olefin elastomers such as an ethylene-propylene block copolymer, and polyurethane elastomers.

As the material for the inventive sealing body, any of the aforesaid thermoplastic elastomers may be used alone or mixed with a thermoplastic resin. Examples of the thermoplastic resin to be mixed with the thermoplastic elastomer include polyethylene and polypropylene.

The hardness of the thermoplastic elastomer to be used in the present invention is adjusted by properly determining the amount of an additive such as an oil to be added or by properly determining the type and amount of the thermoplastic resin to be mixed.

### Cap

As described above, the inventive cap is produced by fitting the inventive sealing body in a generally cylindrical outer frame body of a rigid plastic.

### Outer frame body

Examples of the rigid plastic for the outer frame body include various plastics including polyolefins such as polyethylene, polypropylene and poly-4-methylpentene (e.g., available under the trade name of TPX from Mitsui Chemicals Inc.), polycycloolefins such as ethylene-tetracyclododecene copolymer (e.g., available under the registered trademark of APEL from Mitsui Chemicals Inc.), an acrylonitrile-butadiene-styrene copolymer (ABS), polyesters such as polyethylene naphthalate (PEN), polyethylene terephthalate (PET) and polyarylate, and benzene series polymers such as polyphenylene sulfide (PPS), which are conventionally used for medical instruments.

Among the aforesaid exemplary rigid plastics for the outer frame body, rigid plastics which are highly compatible to and weldable with a plastic to be used as a material for the mouth part of the medical container are particularly preferred.

### Inner frame body

The inventive cap may include a generally cylindrical inner frame body provided in a cylindrical portion of the outer frame body to fix the sealing body in the cylindrical portion of the outer frame body. A material for the inner frame body is not particularly limited, but any of the aforesaid rigid plastics to be used as the material for the outer frame body is preferably used as the material for the inner frame body.

### Medical container

As described above, the inventive medical container may be: (I) a medical container made of plastic including the inventive cap which is welded to a mouth part of the medical container; or (II) a medical container including the inventive sealing body which tightly seals a mouth part of the medical container.

A method for welding the inventive cap to the mouth part of the medical container (I) is not particularly limited, but a conventionally known welding method may be employed. For example, the rigid plastic outer frame body of the cap is welded to the mouth part of the plastic medical container by heat welding or high-frequency welding.

The medical container (I)'may be provided in a so-called kit form, in which a cylindrical holder having a double ended needle is fixed to the outer frame body of the cap welded to the mouth part of the medical container.

A clamping member to be used for fixing the sealing body to the mouth part of the medical container (II) (e.g., a vial) is not particularly limited, but a conventionally known metal thin film may be used as a material for the clamping member.

### EXAMPLES

The sealing body, the cap and the medical container according to the present invention will hereinafter be described by way of examples thereof.

### Production of sealing body, cap and infusion bottle

### Example 1

### (1) Production of sealing body

A sealing body 10 as shown in Fig. 1 was injection-molded from the following compositions A and B.

Composition A prepared by blending a thermoplastic styrene elastomer, a paraffin process oil and polypropylene in a weight ratio of 100:100:20 and having a JIS A hardness of 35

Composition B prepared by blending a thermoplastic elastomer, a paraffin process oil and polypropylene in a weight ratio of 100:200:20 and having a JIS A hardness of 20

For the compositions A and B, a block copolymer (available under the registered trademark of Tuftec H1272 from Asahi Kasei Corp. and having a number average molecular weight (Mn) of 140,000) having a polystyrene-hydrogenated polybutadiene-polystyrene structure was used as the thermoplastic styrene elastomer. A process oil available under the product number of PW-380 from Idemitsu Kosan Co., Ltd. was used as the paraffin process oil. A polypropylene (available under the product number of J430 from Mitsui Chemicals Inc.) having a low molecular weight was used as the polypropylene.

The sealing body 10 was of a disk shape as shown in Fig. 1, and had a diameter D₁ of 23mm. A needle stick part 11 of the sealing body had a diameter D₂ of 12mm. The ratio of the diameter D₂ of the needle stick part 11 to the diameter D₁ of the sealing body 10 was about 52%. The sealing body 10 had a thickness of 5mm.

### (2) Production of Cap

The aforesaid sealing body 10 was inserted into a cylindrical portion of a cylindrical polyethylene outer frame body 16 from the side of a bottom 15b. An inner frame body 18 is inserted into the outer frame body 16 from the side of the bottom 15b, and a projection provided on an outer peripheral surface of the inner frame body 18 was engaged with a groove formed in the cylindrical portion of the outer frame body 16 to fix the inner frame body to the outer frame body (via an engagement portion 19). Thus, a cap 15 as shown in Fig. 2 was provided.

### (3) Production of infusion bottle

The bottom 15b of the cap 15 was heat welded to a mouth part 21 of an infusion bottle body 22 of polyethylene. Thus, an infusion bottle 29 as shown in Fig. 3 was provided. Distilled water (100mL) was preliminarily filled in the infusion bottle body 22.

### Comparative Example 1

### (1) Production of sealing body

A sealing body was produced in substantially the same manner as the sealing body 10 of Example 1, except that only the composition A was used. The sealing body thus produced had no distinction between a needle stick part and an outer edge part thereof, and had the same outer shape as the sealing body 10 of Example 1.

### (2) Production of cap

The aforesaid sealing body was inserted into a cylindrical portion of a polyethylene outer frame body 16 having the same construction as in Example 1 and fixed to the outer frame body. Thus, a cap was provided. This cap had substantially the same construction as the cap 15 of Example 1 shown in Fig. 2, except that the sealing body was different.

### (3) Production of infusion bottle

The bottom of the cap was heat welded to a mouth part 21 of a polyethylene infusion bottle body 22 having the same construction as in Example 1 . Thus, an infusion bottle was provided. The infusion bottle had substantially the same construction as the infusion bottle of Example 1 shown in Fig. 3, except that the sealing body of the cap was different.

### Comparative Example 2

A sealing body was produced in substantially the same manner as the sealing body 10 of Example 1, except that only the composition B was used. The sealing body thus produced had no distinction between a needle stick part and an outer edge part thereof, and had the same outer shape as the sealingbody 10 of Example 1 . An attempt was made to sequentially produce caps by supplying such sealing bodies to a cap production line. However, the sealing bodies adhered to each other to be jammed up in the line.

### Reference Example

Two types of generally disk-like members each having a thickness of about 2.5mm and a diameter of 23mm were respectively injection-molded from the aforesaid compositionsAandB, and then bonded to each other. Thus, a sealing body of a layer structure having the same outer shape as the sealing body 10 shown in Fig. 1 was provided. An attempt was made to sequentially produce caps by supplying such sealing bodies to a cap production line. However, the sealing bodies adhered to each other to be jammed up in the line.

### Test

The infusion bottles produced in Example 1 and Comparative Example 1 were autoclaved at 104°C for 60 minutes.

The needle stick part of each of the autoclaved infusion bottles (the sealing body in Comparative Examples and Reference Example) was stuck with a plastic hollow needle having a diameter of about 4mm, and then the plastic hollow needle was withdrawn.

In turn, the sticking trace was penetrated again by a 16G metal hollow needle, and air was injected into the infusion bottle through the hollow needle to increase the internal pressure of the infusion bottle. At this time, the sealing body was checked for liquid leakage. After the internal pressure of the infusion bottle reached 0.06MPa, the pressure was maintained at this level for 30 seconds, and then the sealing body was checked for liquid leakage.

As a result, the infusion bottle of Example 1 was free from the liquid leakage when the internal pressure was increased and after the pressure was maintained at the increased level.

On the other hand, 10 out of 300 infusion bottles of Comparative Example 1 thus tested suffered from liquid leakage before the internal pressure reached 0.04MPa.

While the present invention has thus been described by way of the embodiments thereof, these embodiments are merely illustrative, but not limitative of the invention. Variations of the present invention apparent to those skilled in the art are to fall within the scope of the present invention defined by the appended claims.

### INDUSTRIAL APPLICABILITY

As described above, the sealing body, and the cap and the medical container each having the sealing body according to the present invention simultaneously ensure easier sticking with a sticking member and a sufficient sealability during the sticking as well as a sufficient sticking member holding capability and easier handling during the sticking, and are advantageously put in practical use in the field of medical containers such as an infusion bottle, an infusion bag and a vial.

## Claims

1. A sealing body having a generally disk-like shape formed of two or more types of thermoplastic elastomers with different hardnesses,
the sealing body comprising at least a needle stick part extended in axial direction and an outer edge part surrounding the needle stick part,
the outer edge part having a higher hardness than that of the needle stick part.

2. A sealing body as set forth in claim 1, wherein
the outer edge part has a JIS A hardness of 25 to 80, and
the needle stick part has a JIS A hardness of 5 to 30.

3. A sealing body as set forth in claim 1, wherein
a difference in JIS A hardness between the outer edge part and the needle stick part is 5 to 15.

4. A sealing body as set forth in claim 1, wherein
the needle stick part is provided in a center portion of the sealing body and has a cylindrical shape, and
the needle stick part has a diameter which is 40 to 70% of a diameter of the entire sealing body.

5. A sealing body as set forth in claim 1, wherein
the needle stick part includes two to five needle stick parts.

6. A sealing body as set forth in claim 1, having a thickness of 3 to 10mm and a diameter of 10 to 40mm.

7. A cap comprising a sealing body having a generally disk-like shape formed of two or more types of thermoplastic elastomers with different hardnesses,
the sealing body comprising at least a needle stick part extended in axial direction and an outer edge part surrounding the needle stick part and having a higher hardness than that of the needle stick part,
wherein the sealing body is tightly fitted in an outer frame body having a generally cylindrical shape formed of a rigid plastic.

8. A medical container made of plastic comprising a cap which is welded to a mouth part of the medical container,
the cap comprising a sealing body having a generally disk-like shape formed of two or more types of thermoplastic elastomers with different hardnesses,
the sealing body comprising at least a needle stick part extended in axial direction and an outer edge part surrounding the needle stick part and having a higher hardness than that of the needle stick part,
wherein the sealing body is tightly fitted in an outer frame body having a generally cylindrical shape formed of a rigid plastic.

9. A medical container comprising a mouth part, wherein the mouth part is tightly sealed by a sealing body having a generally disk-like shape formed of two or more types of thermoplastic elastomers with different hardnesses,
the sealing body comprising at least a needle stick part extended in axial direction and an outer edge part surrounding the needle stick part and having a higher hardness than that of the needle stick part.
